(19) Europäisches Patentamt
European Patent Office
Office européen des brevets

(11) Veröffentlichungsnummer: **0 094 538**
**A2**

(12) **EUROPÄISCHE PATENTANMELDUNG**

(21) Anmeldenummer: **83104269.2**

(22) Anmeldetag: **02.05.83**

(51) Int. Cl.$^3$: **C 07 D 251/50**
C 07 D 251/44, A 01 N 43/70

(30) Priorität: **14.05.82 DE 3218201**

(43) Veröffentlichungstag der Anmeldung:
**23.11.83 Patentblatt 83/47**

(84) Benannte Vertragsstaaten:
**AT BE CH DE FR GB IT LI**

(71) Anmelder: **BAYER AG**
**Zentralbereich Patente, Marken und Lizenzen**
**D-5090 Leverkusen 1, Bayerwerk(DE)**

(72) Erfinder: **Kühle, Engelbert, Dr.**
**von Bodelschwingh-Strasse 42**
**D-5060 Bergisch Gladbach 2(DE)**

(72) Erfinder: **Baasner, Bernd, Dr.**
**Mozartstrasse 41**
**D-5090 Leverkusen(DE)**

(72) Erfinder: **Hagemann, Hermann, Dr.**
**Kandinsky-Strasse 52**
**D-5090 Leverkusen(DE)**

(72) Erfinder: **Eue, Ludwig, Dr.**
**Paul-Klee-Strasse 36**
**D-5090 Leverkusen(DE)**

(72) Erfinder: **Schmidt, Robert R., Dr.**
**In Waldwinkel 110**
**D-5060 Bergisch Gladbach 2(DE)**

(54) **Fluorhaltige 4,6-Diamino-s-triazine, Verfahren und neue Zwischenprodukte zu ihrer Herstellung und ihre Verwendung als Herbizide.**

(57) Neue, fluorhaltige 4,6-Diamino-s-triazine der allgemeinen Formel

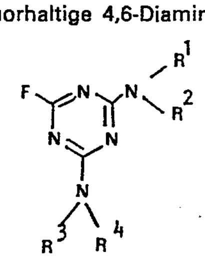

(I)

worin

$R^1$, $R^2$ und $R^3$ gleich oder verschieden sind und für Wasserstoff und/oder einen gesättigten oder ungesättigten aliphatischen Rest stehen und

$R^4$ für einen Fluoralkyl- oder Fluorchloralkylrest steht, ein Verfahren und neue Zwischenprodukte zu ihrer Herstellung und ihre Verwendung als Herbizide.

EP 0 094 538 A2

BAYER AKTIENGESELLSCHAFT 5090 Leverkusen, Bayerwerk
Zentralbereich
Patente, Marken und Lizenzen Bi/Th/c
Ia

# Fluorhaltige 4,6-Diamino-s-triazine, Verfahren und neue Zwischenprodukte zu ihrer Herstellung und ihre Verwendung als Herbizide

Die vorliegende Erfindung betrifft neue fluorhaltige - gleichzeitig kern- und seitenkettenfluorierte - s-Triazinderivate, Verfahren und neue Zwischenprodukte zu ihrer Herstellung sowie ihre Verwendung als Herbizide.

Es ist bereits seit langem bekannt, daß bestimmte s-Triazinderivate als Herbizide eingesetzt werden können. Besondere praktische Bedeutung hat z.B. das 2-Chlor-4-ethylamino-6-isopropylamino-s-triazin (Atrazin) erlangt, welches bekanntermaßen für die Unkrautbekämpfung in Maiskulturen verwendet werden kann (vgl. z.B. "Chemie der Pflanzenschutz- und Schädlingsbekämpfungsmittel", Band 5 - Herbizide, herausgegeben von R.Wegler, Springer-Verlag Berlin, Heidelberg, New-York, 1977, Seiten 336 - 352).

Atrazin ist aber gegen bestimmte Schadgräser, z.B. Alopecurus, Digitaria, Setaria, Poa etc. nicht voll wirksam.

Es wurden nun neue fluorhaltige 4,6-Diamino-s-triazine der allgemeinen Formel

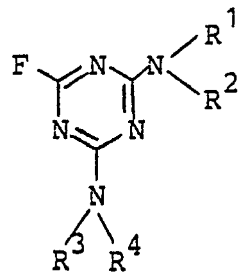

(I),

worin

$R^1$, $R^2$ und $R^3$ gleich oder verschieden sind und für Wasserstoff und/oder einen gesättigten oder ungesättigten aliphatischen Rest stehen und

$R^4$ für einen Fluoralkyl- oder Fluorchloralkylrest steht,

aufgefunden.

Weiterhin wurde gefunden, daß man die s-Triazinderivate der allgemeinen Formel (I) erhält, wenn man 2,4-Difluor-6-(fluoralkylamino)-s-triazine der allgemeinen Formel II

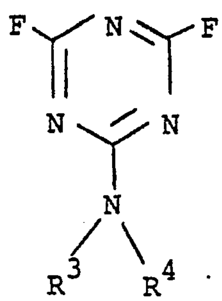

(II),

worin

$R^3$ und $R^4$ die oben angegebene Bedeutung haben,

mit einem Amin der Formel III

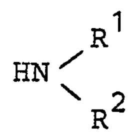

(III),

worin

$R^1$ und $R^2$ die oben angegebene Bedeutung haben,

in Gegenwart eines säurebindenden Mittels und gegebenenfalls in Gegenwart eines Verdünnungsmittels umsetzt (Verfahren A).

Außerdem wurde gefunden, daß die neuen fluorhaltigen s-Triazinderivate der allgemeinen Formel (I) starke herbizide Eigenschaften aufweisen.

Überraschenderweise haben die erfindungsgemäßen Wirkstoffe eine bessere selektive Wirkung und ein breiteres Wirkungsspektrum als die vorbekannten Triazine. Insbesondere hat sich gezeigt, daß die erfindungsgemäßen Wirkstoffe gegen bestimmte Schadgräser erheblich besser wirksam sind als das vorbekannte Atrazin.

Von den erfindungsgemäßen Triazinen der Formel (I) sind bevorzugt diejenigen, in denen $R^1$, $R^2$ und $R^3$ jeweils unabhängig voneinander für Wasserstoff, Alkyl mit 1 - 6 C-Atomen oder Alkenyl mit 3 - 6 C-Atomen stehen und $R^4$ für einen Fluoralkylrest mit 1 - 8 C-Atomen und 1 - 9 F-Atomen oder einen Fluorchloralkylrest mit 1 - 8 C-Atomen und bis zu insgesamt 9 Fluor- und Chloratomen steht.

Verwendet man gemäß Verfahren A beispielsweise 2,4-Difluor-6-(1-methyl-2,2,2-trifluorethylamino)-s-triazin und Ethylamin als Ausgangsstoffe, so kann der Reaktionsablauf durch das folgende Formelschema wiedergegeben werden:

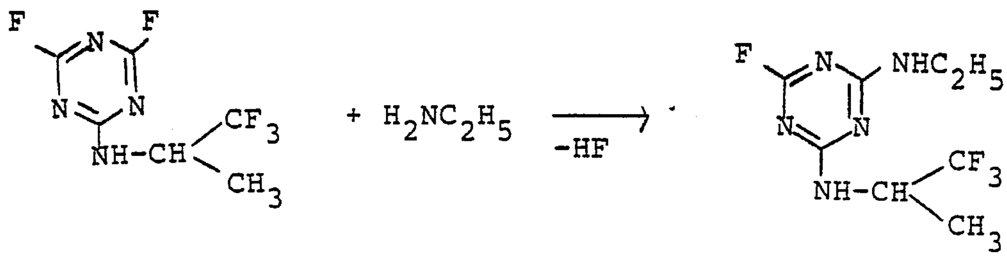


Die nach Verfahren A als Ausgangsverbindungen verwendeten 2,4-Difluor-6-(fluor-alkylamino)-s-triazine der Formel (II) sind neu. Sie können hergestellt werden, indem man Cyanurfluorid der Formel IV

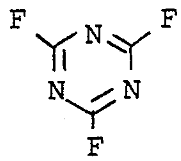

(IV)

mit fluorhaltigen primären und sekundären Aminen der Formel V

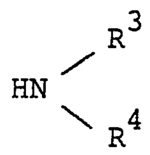

(V)

worin

$R^3$ und $R^4$ die oben angegebene Bedeutung haben,

in Gegenwart eines säurebindenden Mittels und gegebenenfalls in Gegenwart eines Verdünnungsmittels umsetzt (Verfahren B).

Verwendet man gemäß Verfahren B Cyanurfluorid und beispielsweise 1-Methyl-2,2,2-trifluor-ethylamin als Ausgangsstoffe, so läßt sich der Reaktionsablauf durch das folgende Formelschema wiedergeben:

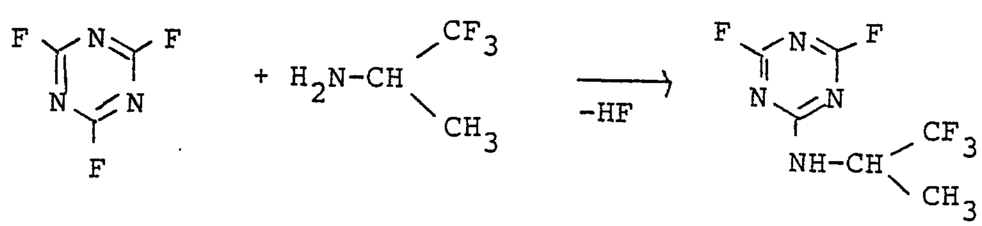


In den Formeln (V) bzw. (II) steht $R^3$ vorzugsweise für Wasserstoff, Alkyl mit 1 - 6 C-Atomen oder Alkenyl mit 3 - 6 C-Atomen. $R^4$ steht in diesen Formeln vorzugsweise für geradkettiges oder verzweigtes Fluoralkyl mit 1 - 8 C-Atomen und 1 - 9 F-Atomen oder für geradkettiges oder

verzweigtes Fluorchloralkyl mit 1 - 8 C-Atomen und bis zu insgesamt 9 Fluor- und Chlor-Atomen.

Besonders bevorzugt sind solche Verbindungen der Formeln (V) bzw. (II), in denen $R^4$ für geradkettiges oder verzweigtes Fluoralkyl mit 1 - 6 C-Atomen und 1 - 7 F-Atomen oder für geradkettiges oder verzweigtes Fluorchloralkyl mit 1 - 6 C-Atomen und bis zu insgesamt 7 Fluor- und Chloratomen steht.

Speziell genannt seien solche Verbindungen der Formel (V) bzw. (II), in denen $R^4$ für 2,2,2-Trifluorethyl, Pentafluorethyl, 3,3,3-Trifluorpropyl, 2,2,2-Trifluor-1-methyl-ethyl, 4,4,4-Trifluor-n-butyl, 1-(Trifluormethyl)-n-propyl, 1-(Trifluormethyl)-n-butyl, 1-(Trifluormethyl)-n-pentyl, 1-(Trifluormethyl)-2-methylpropyl, 3-Fluor-3,3-dichlor-n-propyl, 3-Fluor-3-chlor-n-propyl, 2,2-Difluorpropyl oder für 2,3,3,3-Tetrafluor-2-chlor-propyl steht.

Die nach Verfahren A weiterhin als Ausgangsverbindungen zu verwendenden Amine sind durch Formel (III) allgemein definiert. In dieser Formel stehen $R^1$ und $R^2$ vorzugsweise für Wasserstoff, Alkyl mit 1 - 6 C-Atomen oder Alkenyl mit 3 - 6 C-Atomen.

Die Amine der Formel (III) sind bekannt. Geeignete Vertreter sind Ammoniak, Methyl-, Ethyl-, n-Propyl-, Isopropyl-, n-Butyl-, Isobutyl-, tert.-Butyl-, Hexyl-, Allyl-, Diethyl-, Diallyl- und Dibutylamin.

Das bei Verfahren B einzusetzende Cyanurfluorid (IV) ist bekannt und kann aus Cyanurchlorid durch Umsetzung mit Fluorierungsmitteln, z.B. mit Kaliumfluorid, hergestellt werden (vgl. Houben-Weyl, Methoden der Organ. Chemie, 4. Auflage (1962), Band V/3, Seite 165; Georg Thieme Verlag, Stuttgart).

Die weiterhin bei Verfahren B einzusetzenden fluorhaltigen Amine der Formel (V) sind teilweise bekannt (vgl. J. Org. Chem. 24, S 1256 - 1259 (1959); J. Chem. Soc. 1954, S. 366 - 374; J. Org. Chem. 27, S. 1406 - 1409 (1962); J. Med. Chem. 22, S. 1130 - 1133 (1979); Izvest. Akad. Nauk. SSSR Ser. Khim. 1966, S. 1518 - 1923 (engl.); US-PS 3 908 012; US-PS 3 960 949 und DE-OS 2 117 015).

Die bisher noch nicht im einzelnen beschriebenen fluorierten Amine der Formel (V) lassen sich nach im Prinzip bekannten Methoden herstellen.

So erhält man zum Beispiel diejenigen fluorierten Amine der Formel Va

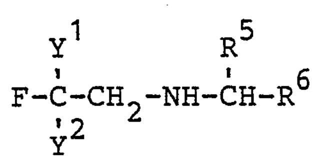

(Va)

in welcher

$Y^1$ für Wasserstoff, Fluor oder Chlor steht,
$Y^2$ für Wasserstoff, Fluor oder Chlor steht,
$R^5$ für Alkyl steht und
$R^6$ für Wasserstoff oder Alkyl steht,

indem man fluorierte Azomethine der Formel VI

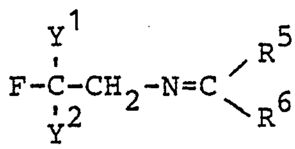

(VI)

in welcher

$Y^1$, $Y^2$, $R^5$ und $R^6$ die oben angegebene Bedeutung haben,

mit Wasserstoff unter einem Druck von 3 bis 15 bar in Gegenwart eines Katalysatos, wie Platin auf Kohle, Palladium auf Kohle oder Raney-Nickel, sowie in Gegenwart eines Verdünnungsmittels, zum Beispiel eines Alkohols wie Methanol oder Ethanol, oder eines Ethers wie Dioxan, bei Temperaturen zwischen 0°C und 60°C, vorzugsweise zwischen 10°C und 50°C hydriert.

In der Formel (VI) steht $Y^1$ vorzugsweise für Wasserstoff, Fluor oder Chlor. $Y^2$ steht ebenfalls vorzugsweise für Wasserstoff, Fluor oder Chlor. $R^5$ steht vorzugsweise für Alkyl mit 1 bis 4 Kohlenstoffatomen, und $R^6$ steht vorzugsweise für Wasserstoff oder Alkyl mit 1 bis 4 Kohlenstoffatomen.

Die bei der Herstellung der fluorierten Amine der Formel (Va) nach dem oben beschriebenen Verfahren als Ausgangsstoffe benötigten Azomethine der Formel (VI)

sind bisher noch nicht bekannt. Sie lassen sich jedoch herstellen, indem man Amine der Formel VII

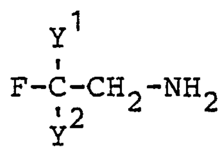

(VII)

in welcher

$Y^1$ und $Y^2$ die oben angegebene Bedeutung haben,

mit Carbonylverbindungen der Formel VIII

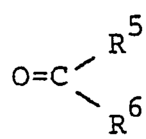

(VIII)

in welcher

$R^5$ und $R^6$ die oben angegebene Bedeutung haben,

gegebenenfalls in Gegenwart eines Verdünnungsmittels, wie Pentan, Hexan, Cyclohexan, Chloroform, Tetrachlorkohlenstoff, Benzol, Toluol, Xylol, Chlorbenzol, Diethylether, Tetrahydrofuran oder Dioxan, bei Temperaturen zwischen -20°C und +60°C, vorzugsweise zwischen 0°C und +40°C umsetzt.

Die bei der Synthese der fluorierten Azomethine der Formel (VI) nach dem obigen Verfahren als Ausgangsstoffe benötigten Amine der Formel (VII) und Carbonylverbindungen der Formel (VIII) sind bekannt oder lassen sich nach im Prinzip bekannten Methoden herstellen (vgl. hierzu DE-OS 30 18 030).

Das als Ausgangsverbindung besonders interessante 1-Methyl-2,2,2-trifluorethylamin kann zweckmäßigerweise ausgehend von 1,1-Difluorpropen über 1-Methyl-2,2,2-trifluornitroethan und dessen katalytische Hydrierung - gemäß folgendem Formelschema - hergestellt werden (vgl. zur Methode: Izvest. Akad. Nauk SSSF, Ser. Khim. 1963, S. 1794 ff (engl.); ferner Tetrahedron 26, S. 5737 - 5743 (1970)):

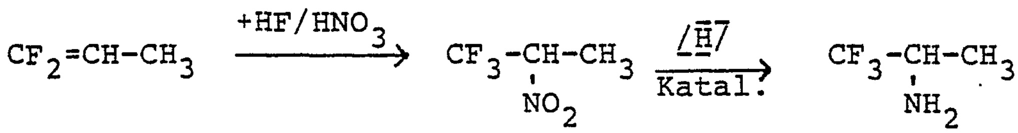


Bei der Durchführung der konjugierten Addition von HF und $HNO_3$ an 1,1-Difluorpropen setzt man die Salpetersäure bevorzugt in äquimolarer Menge ein (wobei ein Überschuß möglich ist ); Fluorwasserstoff wird in überstöchiometrischen Mengen eingesetzt und dient gleichzeitig als Lösungsmittel. Man erhält die Nitroverbindung in Ausbeuten bis zu 86 % der Theorie. - Bei der anschließenden katalytischen Hydrierung können alle üblichen Edelmetallkatalysatoren wie Pd oder Pt, aber auch Raney-Nickel verwendet werden. Ferner kann die Reduktion auch mittels nascierendem Wasserstoff, z.B. mit dem System

$Fe/H^+$ - mit oder ohne Zusatz von Basen wie $MgCO_3$ oder $Na_2CO_3$ als Säurefänger - durchgeführt werden. Als Lösungs- bzw. Verdünnungsmittel sind Alkohole wie Methanol oder Ethanol, Ether wie z.B. Diisopropylether sowie Wasser geeignet (vgl. Beispielteil).

Die Reaktionstemperaturen können bei den beiden oben beschriebenen erfindungsgemäßen Verfahren A und B in einem gewissen Bereich variiert werden. Im allgemeinen arbeitet man bei Verfahren A zwischen 0 und 50°C, (vorzugsweise zwischen 20 und 45°C) und bei Verfahren B im allgemeinen zwischen -20 und +10°C (vorzugsweise zwischen -10° und 0°C).

Die nachfolgenden allgemeinen Angaben betreffend die Molverhältnisse, Verdünnungsmittel, Säurebindemittel und Aufarbeitung beziehen sich jeweils auf die beiden Verfahren A und B.

Bei der Durchführung der erfindungsgemäßen Verfahren setzt man die Ausgangsstoffe und gegebenenfalls die Säurebindemittel in etwa äquimolaren Mengen ein.

Die Umsetzungen werden bevorzugt in Gegenwart eines Verdünnungsmittels durchgeführt. Als Verdünnungsmittel kommen alle inerten organischen Lösungsmittel in Frage. Hierzu gehören vorzugsweise Kohlenwasserstoffe, wie Benzol und Xylol, chlorierte Kohlenwasserstoffe, wie Chlorbenzol und Tetrachlorkohlenstoff; Ketone, wie Aceton und Methylethylketon; Ether, wie Tetrahydrofuran

und Dioxan; Amide, wie Dimethylformamid. Die Umsetzungen können zum Teil auch im wäßrigen Medium durchgeführt werden.

Als Säurebinder können alle üblichen Säurebindungsmittel verwendet werden. Hierzu gehören vorzugsweise die tertiären Amine, wie Trimethylamin, Triethylamin und Pyridin, Alkalihydroxide, wie Natriumhydroxid und Kaliumhydroxid sowie auch Alkalicarbonate und -hydrogencarbonate, wie Natriumcarbonat, Kaliumcarbonat oder Natriumhydrogencarbonat. Bei Verfahren A kann auch ein molarer Überschuß an Amin der Formel (III) als Säurebinder dienen.

Die Aufarbeitung und Isolierung der Reaktionsprodukte erfolgt in üblicher Weise. Bei Zugabe von Wasser zum Reaktionsgemisch fallen die Reaktionsprodukte im allgemeinen bereits kristallin aus, falls Lösungsmittel verwendet worden sind, die sich mit Wasser mischen.

Die erfindungsgemäßen Wirkstoffe beeinflussen das Pflanzenwachstum und können deshalb als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dicotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca; Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen wirken sowohl gegen dikotyle Pflanzen als auch gegen monokotyle Pflanzen, insbesondere gegen Gräser. Gerade in der Wirkung gegen Gräser, z.B. Alopecurus, Avena fatua, Digitaria und Setaria, sind die neuen Wirkstoffe den bekannten herbiziden Triazinen überlegen. Ein selektiver Einsatz der erfindungsgemäßen Wirkstoffe ist in verschiedeneh Kulturen möglich, z.B. in Getreide, Baumwolle und Zwiebeln. Die Wirkstoffe sind insbesondere für Mais sehr gut verträglich; sie können daher mit besonderem Vorteil als selektive Maisherbizide eingesetzt werden, wobei sie wegen ihrer wesentlich besseren Wirkung insbesondere gegen Schadgräser dem vorbekannten Maisherbizid Atrazin überlegen sind.

Die Wirkstoffe können in die üblichen Formulierungen übergeführt werden, wie Lösungen, Emulsionen, Spritzpulver, Suspensionen, Pulver, Stäubemittel, Pasten,

lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. natürliche Gesteinsmehle, wie Kaoline, Tonerden, Talkum, Kreide, Quarz, Attapulgit, Montmorillonit oder

Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sephiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugenden Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methycellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden - z.B. Chloracetaniliden wie Alachlor oder Metolachlor - zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich. Für bestimmte Anwendungszwecke kann es ferner vorteilhaft sein, in die Formulierungen als weitere Zusatzstoffe pflanzenverträgliche mineralische oder vegetabilische Öle (z.B. das Handelspräparat "Sun Oil 11E") oder Ammoniumsalze wie z.B. Ammoniumsulfat oder Ammoniumrhodanid aufzunehmen.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen oder Stäuben.

Die erfindungsgemäßen Wirkstoffe können sowohl vor als auch nach dem Auflaufen der Pflanzen appliziert werden.

Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,1 und 10 kg Wirkstoff pro ha, vorzugsweise zwischen 0,2 und 5 kg/ha.

Die nachfolgenden Beispiele dienen zur weiteren Erläuterung der Erfindung.

Herstellungsbeispiele

A) Triazine der Formel (I) - nach Verfahren A

Beispiel A-1

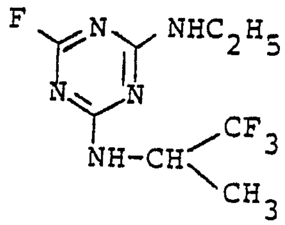

(A-1)

10 g (0,046 Mol) 2,4-Difluor-6-(1-methyl-2,2,2-trifluorethylamino)-s-triazin werden in 100 ml Toluol gelöst und bei 10 - 15°C unter Kühlung mit 10 ml (0,11 Mol) einer 50 %igen wäßrigen Ethylaminlösung versetzt. Hierbei fällt das Reaktionsprodukt sofort aus. Man säuert die Lösung mit Salzsäure an, saugt ab, wäscht mit Wasser und trocknet. Ausbeute 10 g (≙ 89 % d.Th.) 2-Fluor-4-ethylamino-6-(1-methyl-2,2,2-trifluorethylamino)-s-triazin, Schmelzpunkt 175 - 177°C.

In analoger Weise erhält man die in der nachfolgenden Tabelle 1 aufgeführten Verbindungen der Formel (I):

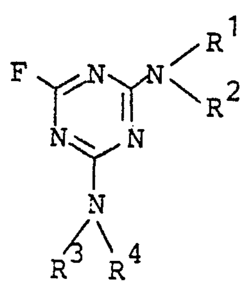

(I)

Tabelle 1

| Beispiel Nr. | $R^1$ | $R^2$ | $R^3$ | $R^4$ | Schmelzpunkt |
|---|---|---|---|---|---|
| A-2 | $CH_3$ | H | H | $CF_3-\underset{CH_3}{\underset{\vert}{CH}}-$ | 177°C |
| A-3 | $i-C_3H_7$ | H | H | $CF_3-\underset{CH_3}{\underset{\vert}{CH}}-$ | 207°C |
| A-4 | $n-C_3H_7$ | H | H | $CF_3-\underset{CH_3}{\underset{\vert}{CH}}-$ | 183°C |
| A-5 | $i-C_4H_9$ | H | H | $CF_3-\underset{CH_3}{\underset{\vert}{CH}}-$ | 191°C |
| A-6 | $n-C_4H_9$ | H | H | $CF_3-\underset{CH_3}{\underset{\vert}{CH}}-$ | 154°C |
| A-7 | $C_2H_5$ | H | H | $CF_3-CH_2-CH_2-$ | 186°C |
| A-8 | $C_2H_5$ | H | H | $CF_3-CH_2-$ | 230°C |
| A-9 | $CH_3$ | H | H | $CFCl_2-CH_2-CH_2-$ | 194°C |
| A-10 | $C_2H_5$ | H | H | $CFCl_2-CH_2-CH_2-$ | 183°C |
| A-11 | $CH_3$ | H | H | $CF_2Cl-\underset{CH_3}{\underset{\vert}{CH}}-CH_2-$ | 196°C |
| A-12 | $C_2H_5$ | H | H | $CF_2Cl-\underset{CH_3}{\underset{\vert}{CH}}-CH_2-$ | 184°C |

Tabelle 1 (Fortsetzung)

| Beispiel Nr. | $R^1$ | $R^2$ | $R^3$ | $R^4$ | Schmelzpunkt |
|---|---|---|---|---|---|
| A-13 | $CH_3$ | H | H | $CFCl_2-CH-CH_2-$<br>$CH_3$ | 200 °C |
| A-14 | $C_2H_5$ | H | H | $CFCl_2-CH-CH_2-$<br>$CH_3$ | 175 °C |
| A-15 | i-$C_3H_7$ | H | H | $CF_3-CH_2-CH_2-$ | 185 °C |
| A-16 | i-$C_3H_7$ | H | H | $CF_3-CH_2-$ | 201 °C |
| A-17 | n-$C_3H_7$ | H | H | $CF_3-CH_2-$ | 224 °C |
| A-18 | i-$C_3H_7$ | H | H | $CFCl_2-CH_2-CH_2-$ | 190 °C |
| A-19 | i-$C_3H_7$ | H | H | $CF_2Cl-CH-CH_2-$<br>$CH_3$ | 176 °C |
| A-20 | i-$C_3H_7$ | H | H | $CFCl_2-CH-CH_2-$<br>$CH_3$ | 185 °C |

B) Triazine der Formel (II)- nach Verfahren B

Beispiel B-1

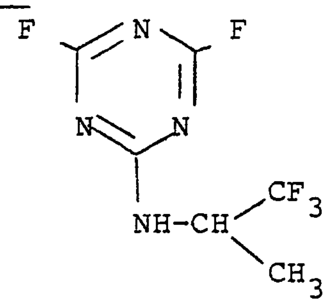

(B-1)

13,5 g (0,1 Mol) Cyanurfluorid werden in 100 ml Methylenchlorid gelöst. Bei -5°C bis 0°C läßt man hierzu eine Lösung von 11,5 g (ca. 0,1 Mol) 1-Methyl-2,2,2-trifluorethylamin in 50 ml Methylenchlorid zutropfen. Man rührt etwa 15 Minuten nach und tropft im gleichen Temperaturbereich eine Lösung von 9 g (0,108 Mol) Natriumhydrogencarbonat in 100 ml Wasser zu. Man läßt die Temperatur auf 10°C ansteigen, trennt die beiden Schichten voneinander und engt die getrocknete Methylenchloridlösung ein.

Ausbeute: 20 g (≙ 92% d.Th.) 2,4-Difluor-6-(1-methyl-2,2,2-trifluor-ethylamino)-s-triazin; Schmelzpunkt 72 - 74°C.

In analoger Weise lassen sich die in der nachfolgenden Tabelle 2 aufgeführten Verbindungen der Formel (II) herstellen:

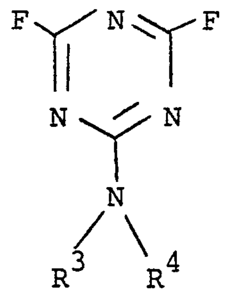

(II)

Tabelle 2

| Beispiel Nr. | $R^3$ | $R^4$ | Schmelzpunkt |
|---|---|---|---|
| B-2 | H | $CF_3-CH_2-CH_2-$ | 53 - 56°C |
| B-3 | H | $CF_3-CH_2-$ | 113 - 115°C |
| B-4 | H | $CFCl_2-CH_2-CH_2-$ | Öl |
| B-5 | H | $CF_2Cl-CH(CH_3)-CH_2-$ | Öl |
| B-6 | H | $CFCl_2-CH(CH_3)-CH_2-$ | Öl |

C) Fluorhaltige Amine der Formel (V)

Beispiel C-1

$$CF_3-\underset{NH_2}{CH}-CH_3$$

(a) $$CF_2=CH-CH_3 + HNO_3 + HF \longrightarrow CF_3-\underset{NO_2}{CH}-CH_3$$

1400 ml Fluorwasserstoff (wasserfrei) und 320 g Salpetersäure (konz.) werden in einem Stahlrührautoklaven vorgelegt und auf -30° bis -40°C gekühlt. Bei dieser Temperatur werden 390 g (5 Mol) 1,1-Difluorpropen eingeleitet. Man läßt auf Raumtemperatur aufwärmen, rührt 6 Stunden nach und gießt das Reaktionsgemisch dann auf 2 kg Eis. Die wäßrige Phase wird dreimal mit je 250 ml Dichlormethan extrahiert, die vereinigten organischen Phasen werden mit $NaHCO_3$-Lösung und Wasser neutral gewaschen, mit $MgSO_4$ getrocknet und destilliert. Nach Abtrennen des Lösungsmittels und eines Vorlaufes (25 g; Siedebereich: 43 - 98°C) erhält man 615 g (≙ 86 % d.Th.) 1-Methyl-2,2,2-trifluornitroethan /≡ 1-(Trifluormethyl)-nitroethan/ ; Siedepunkt (Kp) 98 - 100°C; $n_D^{20}$: 1,3380.

(b) 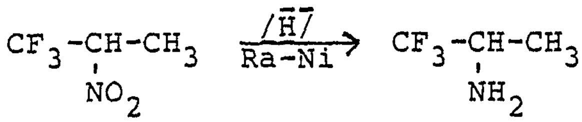


715 g (5 Mol) 1-Methyl-2,2,2-trifluor-nitroethan werden in 3 Liter Methanol mit 30 g Raney-Nickel bei einer Temperatur von 30 - 40°C und einem Druck von 40 bar hydriert. Nach dem Abfiltrieren des Katalysators wird mit konz. Salzsäure angesäuert und die flüchtigen Bestandteile werden abdestilliert. Zum Rückstand gibt man 600 g 50 %ige Natronlauge und destilliert das Reaktionsprodukt ab. Man erhält 520 g (≙ 92 % d.Th.) 1-Methyl-2,2,2-trifluor-ethylamin /≙ 1-(Trifluormethyl)-ethylamin7; Siedepunkt 47 - 48°C; $n_D^{20}$: 1,3215.

<u>Beispiel C-2</u>

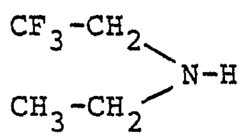

(a) $CF_3-CH_2-NH_2 + CH_3CHO \longrightarrow CF_3-CH_2-N{=}CH-CH_3$

Zu 99 g (1 Mol) 2,2,2-Trifluorethylamin werden innerhalb von 60 Minuten unter Eiskühlung 44 g (1 Mol) frisch destillierter Acetaldehyd zugetropft. Man läßt 1 Stunde nachrühren, gibt 15 g festes Kaliumhydroxid hinzu und trennt die Phasen. Die organische Phase wird über etwa

5 g festem Kaliumhydroxid getrocknet und anschließend über eine Kolonne destilliert. Nach einem Vorlauf (20 g; Kp: 18 - 42°C), der aus nicht umgesetztem Amin und Acetaldehyd besteht, erhält man 77,5 g (62 % der Theorie) an Ethyliden-2,2,2-trifluorethyl-amin.

Kp = 73 - 74°C

$n_D^{20}$ = 1,3415

(b) 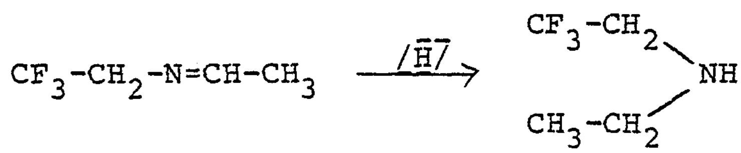


125 g (1 Mol) Ethyliden-2,2,2-trifluorethylamin werden in 250 ml absolutem Ethanol gelöst, mit 4 g Platin auf Kohle (5 %ig) versetzt und bei 30°C unter einem Wasserstoffdruck von 10 bar 90 Minuten lang hydriert. Nach dem Abfiltrieren des Katalysators wird mit konzentrierter Salzsäure angesäuert und unter vermindertem Druck bis zur Trockne eingeengt. Das dabei anfallende Produkt wird mit 100 ml 50 %iger wäßriger Natronlauge versetzt und destilliert. Man erhält auf diese Weise 83 g (65 % der Theorie) an 2,2,2-Trifluor-ethyl-N-ethylamin.

Kp = 61 - 62°C

$n_D^{20}$ = 1,3335

In analoger Weise werden auch die folgenden fluorierten Amine erhalten:

## Beispiel C-3

(a) Zwischenprodukt:

$CF_3-CH_2-N=CH-CH_2CH_3$

Ausbeute: 64 % der Theorie
Kp = 93 - 95°C

(b) 2,2,2-Trifluor-ethyl-N-n-propylamin

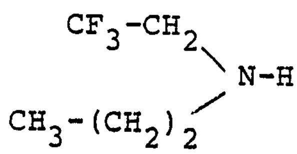

Ausbeute: 66 % der Theorie
$n_D^{20}$ = 1,3462
Kp = 74°C

## Beispiel C-4

(a) Zwischenprodukt:

$CF_3-CH_2-N=CH-CH_2-CH_2-CH_3$

Ausbeute: 72 % der Theorie
Kp = 116 - 118°C

(b) 2,2,2-Trifluor-ethyl-N-n-butylamin

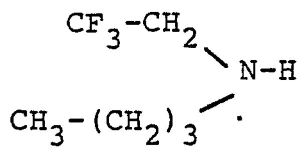

Ausbeute: 72 % der Theorie

$n_D^{20}$ = 1,3590

Kp = 84°C

## Verwendungsbeispiele

### Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigen z.B. die folgenden Verbindungen der Herstellungsbeispiele eine ausgezeichnete Wirksamkeit: Nr. A-1.

Patentansprüche

1) Fluorhaltige 4,6-Diamino-s-triazine der allgemeinen Formel I

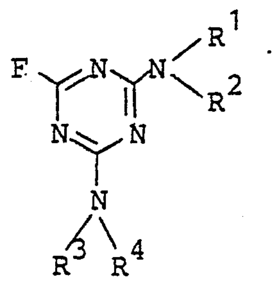

(I),

worin

$R^1$, $R^2$ und $R^3$ gleich oder verschieden sind und für Wasserstoff und/oder einen gesättigten oder ungesättigten aliphatischen Rest stehen und

$R^4$ für einen Fluoralkyl- oder Fluorchloralkylrest steht.

2) Fluorhaltige 4,6-Diamino-s-triazine der allgemeinen Formel (I) gemäß Anspruch 1, worin

$R^1$, $R^2$ und $R^3$ jeweils unabhängig voneinander für Wasserstoff, Alkyl mit 1 - 6 C-Atomen oder Alkenyl mit 3 - 6 C-Atomen stehen und

$R^4$ für einen Fluoralkylrest mit 1 - 8 C-Atomen und 1 - 9 F-Atomen oder einen Fluorchloralkylrest mit 1 - 8 C-Atomen und bis zu insgesamt 9 F- und Cl-Atomen steht.

3) 2-Fluor-4-ethylamino-6-(1-methyl-2,2,2-trifluor-ethylamino)-s-triazin der Formel

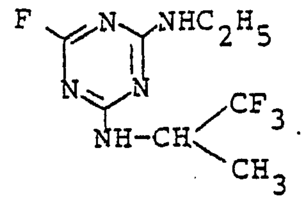

(A-1)

gemäß Anspruch 1.

4) 2-Fluor-4-methylamino-6-(1-methyl-2,2,2-trifluor-ethylamino)-s-triazin der Formel

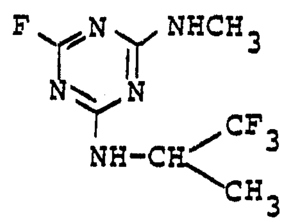

(A-2)

gemäß Anspruch 1.

5) Verfahren zur Herstellung von fluorhaltigen 4,6-Diamino-s-triazinen der allgemeinen Formel I

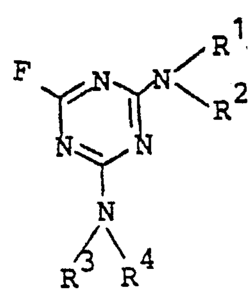

(I),

worin

$R^1$, $R^2$ und $R^3$ gleich oder verschieden sind und für Wasserstoff und/oder einen gesättigten oder ungesättigten aliphatischen Rest stehen und

$R^4$ für einen Fluoralkyl- oder Fluorchloralkylrest steht,

dadurch gekennzeichnet, daß man 2,4-Difluor-6-(fluor-alkylamino)-s-triazine der allgemeinen Formel II

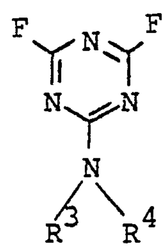

(II),

worin

$R^3$ und $R^4$ die oben angegebene Bedeutung haben,

mit einem Amin der Formel III

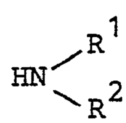

(III),

worin

$R^1$ und $R^2$ die oben angegebene Bedeutung haben,

in Gegenwart eines säurebindenden Mittels und gegebenenfalls in Gegenwart eines Verdünnungsmittels umsetzt.

6) 2,4-Difluor-6-(fluor-alkylamino)-s-triazine der allgemeinen Formel II

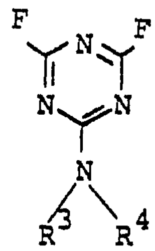

(II),

worin

$R^3$ und $R^4$ die oben angegebene Bedeutung haben.

7) Verfahren zur Herstellung von 2,4-Difluor-6-(fluor-alkylamino)-s-triazinen der allgemeinen Formel II

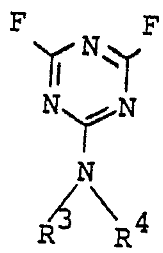

(II),

worin

$R^3$ und $R^4$ die oben angegebene Bedeutung haben,

dadurch gekennzeichnet, daß man Cyanurfluorid der Formel IV

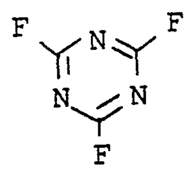

(IV)

mit fluorhaltigen primären und sekundären Aminen der Formel V

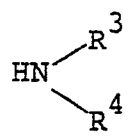

(V)

worin

$R^3$ und $R^4$ die oben angegebene Bedeutung haben,

in Gegenwart eines säurebindenden Mittels und gegebenenfalls in Gegenwart eines Verdünnungsmittels umsetzt.

8) Herbizide Mittel, gekennzeichnet durch einen Gehalt an mindestens einem fluorhaltigen 4,6-Diamino-s-triazin der Formel (I) gemäß Anspruch 1 bzw. 5.

9) Verwendung von fluorhaltigen 4,6-Diamino-s-triazinen der Formel (I) gemäß Anspruch 1 bzw. 5 zur Bekämpfung von Unkräutern.

10) Verfahren zur Herstellung von herbiziden Mitteln, dadurch gekennzeichnet, daß man fluorhaltige 4,6-Diamino-s-triazine der Formel (I) gemäß Anspruch 1 bzw. 5 mit Streckmitteln und/oder oberflächenaktiven Mitteln vermischt.